# EUROPEAN PATENT APPLICATION

(11) **EP 2 799 867 A1**
(43) Date of publication of application: **05.11.2014**
(21) Application number: 13425065.3
(22) Date of filing: 30.04.2013
(51) Int. Cl.: G01N 33/28, G01N 21/55

(54) **Device for real time control of oils and fuels quality in internal combustion engines**

(71) Applicant: Universita'del Salento, 73100 Lecce (IT)
(72) Inventor: De Risi, Arturo, 73100 Lecce (IT); Milanese, Marco, 73010 Arnesano (LE) (IT)
(74) Representative: Bruni, Giovanni

(57) **Abstract**

The present invention relates to a device based on the Surface Plasmon Resonance technique (SPR) for the accurate and in real time diagnostics of the characteristics of the fuel and/or lubricant in internal combustion engines.

## Description

The present invention relates to the development of a sensor for the diagnostics of fuel and/or lubricant characteristics in internal combustion engines. More in detail, the present device, object of the present invention, exploits the technique of the Surface Plasmon Resonance (SPR) in order to analyze accurately and in real time the state of the lubricating oil and/or the fuel characteristics, independently of its history or the type or quantity of pollutant present therein.

Therefore, the aim of the present invention is a double one: on the one hand it aims at the characterization of the lubricating oil in order to manage the oil changes no more according to a prefixed program (normally every 20000-30000 km) but according to the real degradation state of the lubricant; on the other hand it aims at an optimized management of the combustion cycles in the engines, even in "multifuel" ones, according to the specific characteristics of the fuel.

As it is known at the state of the art, the performance of an internal combustion engine depends on many factors and apparatuses, among which the most important one is the lubricating system which has to contain friction and wear of the engine moving portions, besides the corrosion prevention.

Recently considerable attention has been paid to the physical-chemical, mechanical, optic and electrical properties of lubricants and many attempts to develop sensors able to allow monitoring have been made by means of direct and indirect measures.

The conventional maintenance routine of an engine consists in monitoring the lubricant level and in substituting oil according to the distance covered (usually every 20000-30000 km). In the light of the recent government rules on the environmental impact, of the increasing expectations of clients on car performances and of the greater efficiency asked to the engines this approach "based on quantity" is no more sufficient. All these factors drive necessarily towards the real time monitoring of the lubricating conditions so that the substitution of the oil used occurs at the optimal time or when it is observed an unexpected and worrying speed of the degradation of the same.

Recently the applications of the SPR technique for the development of sensors have become always more frequent: study of the optic properties of the metal layers, characterization of the film thickness, measurement of the optical parameters of the organic layers deposited on metal surfaces, adsorbing and desorbing mechanisms involving biomaterials, application of sensing gas and biosensors.

There is a plurality of thematic areas which go from the physical applications to the biological ones. Generally, the optic sensors based on the superficial plasma waves refer to the measurement of the variations of the refraction index or not optic variations able to produce variations in the refraction index. In other words, a variation of the refraction index can be measured by monitoring the wave propagation constant changing of the plasmon surface wave (SPW) after the modification of the characteristic of the light waves interaction with the same SPW. In this context, new possible applications of the SPR technique are introduced in the automotive field where recently the importance of the monitoring systems role has increased becoming one of the most important issues in the new vehicles design.

The idea of the present patent application is to realize a method and a relative device, based on the SPR technique, to be installed in an engine, able to characterize in real time both the engine lubricant (in order to carry out oil changes at the real need moment) and the fuel in even multifuel applications (in order to optimize the combustion cycle).

Therefore, aim of the present invention is a double one:
- characterizing in real time the vehicle lubricating oil in order to manage the oil changes according to the real degradation state of the same, abandoning the logic of the programmed oil change every 20000-30000 km of distance covered (first configuration);
- characterizing in real time the fuel in order to obtain an optimized management of the combustion cycles in internal combustion engines, even of "multifuel" type (second configuration).

In order to reach the prefixed aims, the present invention relates to a method for monitoring in real time working fluids in internal combustion engines (fuels and/or lubricants) based on the SPR physical principle comprising the following steps: emission of an electromagnetic wave in the visible and/or infrared spectrum; detection of an electromagnetic wave reflexed by an optic prismatic coupling - metal film and dielectric, this latter comprising a working fluid; processing of the biochemical parameter values; interaction with the engine control system for signaling possible anomalies or for the request of modifications of the engine management.

The above cited aims are reached also by means of a device for monitoring in real time the working fluids in internal combustion engines (fuels and/or lubricants) based on the SPR physical principle comprising a coupling between an optic system and a metal layer, a signal transduction system between the optic property variation and the variation of the property of interest, an electronic system for said data processing.

These and other advantages will be highlighted in the following detailed description of the present invention, which refers to drawings 1/3 to 3/3 which show some preferred, absolutely not limiting embodiments of the invention. In particular:
- Fig. 1 shows a schematic representation of the known physical principle of the Surface Plasmon Resonance (SPR);
- Fig. 2 shows a schematic representation of a preferred embodiment of the SPR device to be applied to the internal combustion engine;
- Fig. 3 shows the detail of the light beam path from the emission point to the reading point, after the plasmon is stressed;
- Fig. 4 shows graphically the variation of the reflectance detected at a fixed incidence angle of the light beam;
- Fig. 5 shows the functioning scheme of the device in case of real time monitoring of the quality of the fuel in an internal combustion engine.

As yet said, the sensor object of the present invention is based on the known physical principle of the Surface Plasmon Resonance. A plasmon surface is an electromagnetic wave associated to a longitudinal oscillation of the free electrons on the surface of a dielectric medium (as water or air) and a metal. In other words, the SPR is a very sensible technique, which allows little variations of the refraction index to be measured at the interface between a metal and a dielectric medium.

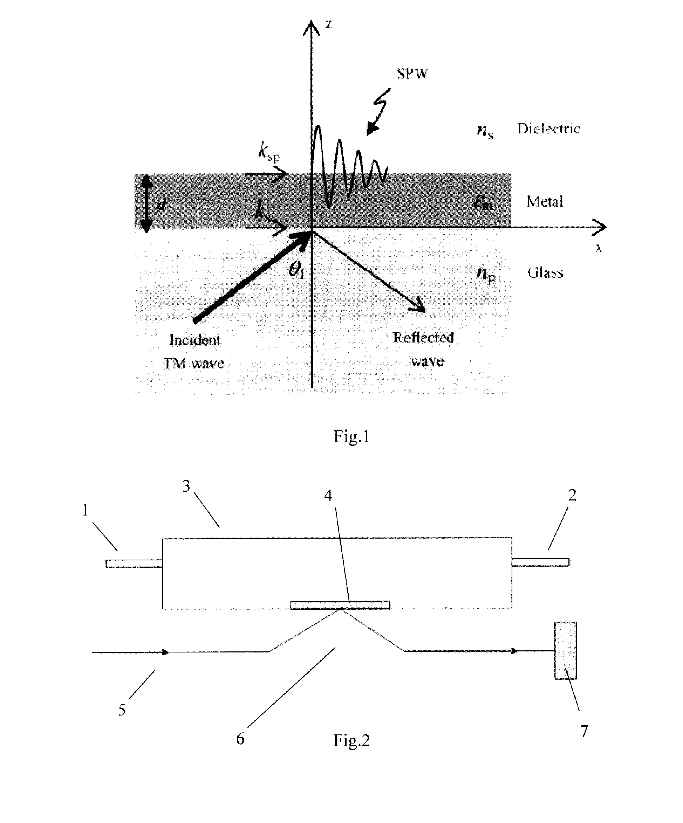

With reference to fig. 1, if the metal layer (d) is sufficiently thin, an incident light radiation, used as probe, will have the possibility to strike a surface of the metal layer, to penetrate the same e to reach the other surface of the layer, exciting a surface plasmon (PS) which is propagated along the metal-dielectric 5 interface. The surface plasmon is nothing but a collective oscillation of free electrons of the metal.

Since the propagation constant of a plasma wave surface is always greater than the one of a light wave in the dielectric, a plasmon wave surface (SPW) at the metal-dielectric plan interface cannot be excited directly by an optic wave coming from the same dielectric. A possible solution to solve this problem is to make the exciting light beam pass through a dielectric with a high refraction index prior to strike the metal surface. There are some technical solutions about how to organize the conditions for exciting the plasmon surface.

An SPR sensor is fundamentally made up of three portions: an optic system which contains a radiation source and an optic structure in which the surface plasmon wave is excited and interrogated; a signal transduction system which puts in relation the optic domain with the biochemical one, and therefore, transforms the changes in quantity of interest in changes of the refraction index; an electronic system supporting the sensor optic-electronic components which allows the data obtained during the SPR interrogation step to be processed.

There are known many configurations of SPR devices among which the most used one to increase the momentum of an optic wave to allow a light wave and a plasmon wave surface (SPW) to be coupled at the metal interface, is the configuration with prismatic coupling which exploits the attenuated total reflection (ATR). Referring to this latter configuration, the Kretschmann geometry is the most suitable in the sensoristic field and provides that a light beam is first propagated through a prism with high refraction index and then totally reflected at the interface with a metal-dielectric wave guide which consists of a thin metal film (having a given permittivity and a given thickness) and a semi-infinite dielectric having refraction index lower than the prismatic one. Generally in SPR sensors based on the prism, there can be carried out measures as for example: measuring the wave intensity of reflexed light; measuring the resonance angle of the incident light wave; measuring the resonance wave length. Moreover, another classification in the SPR sensor field is the one between angular modulation sensors, sensors of wave length, of intensity or phase according to the characteristics which are varied by a surface plasmon. In particular, in the angular interrogation sensors, a monochromatic light wave is used to excite a surface plasmon. The resonance intensity between the incident wave and the surface plasmon is observed at multiple incidence angles, normally using a convergent light beam. The surface plasmon exciting manifests itself with a decreasing in the angular spectrum of the reflexed light, and the incidence angle leading to the resonance is used as sensor output (θₛₚᵣ).

In the experiments based on SPR, the metals more generally used are gold (Au) and silver (Ag) since their resonance is localized in the visible spectrum. The Ag film produces sharper resonance peaks and as a consequence a more sensible measure can be obtained (if on the film surface a protective layer is deposited). On the contrary, even if the Au films produce wider resonance peaks, their high stability makes them ideal for the realization of chemical sensors.

For the gold and silver layers, the fading field in this configuration has a maximum range of about 200 nm normal at the metal surface while the layer has an optimal thickness of about 50 nm. If the metal layer is too thin the plasmon will arrive again inside the primes thus loosing intensity.

If the light incidence angle on a metal film of correct thickness is scanned, at a certain angle, the intensity of the reflected light will be rapidly zero, indicating the resonant coupling with the plasmon surface. The position and width of the resonance angle in this point are very sensible to the surface properties and this makes possible the use of surface plasmon resonance techniques for chemical and biological detections.

Therefore, by exploiting the sensibility of the propagation constant of a plasmon surface with respect to the refraction index, the SPR sensors allow the variations of the refraction index produced by a change in the propagation constant of the plasmon wave to be measured.

In this way, the technique used in the present patent allows the physical properties of a liquid (in the specific case the lubricant or the fuel) to be monitored by means of an analysis of the resonance angle measured with an angular precision of 0.0005°. In particular the Kretschmann geometry of the ATR method was found to be very suitable for detecting a wide range of phenomena and has become the most widely used geometry in the SPR sensors and biosensors.

In fig. 2 it is shown the device 10 comprising the coupling prism according to the Kretschmann geometry for the application to an internal combustion engine. Said device is made up of: an inlet 1 and output section 2 of the fluid to be analyzed (lubricating oil or fuel), an analyzer 3, a metal surface 4, the optic prism 6 according to the geometric configuration chosen and a receiver 7 for detecting the reflected light beam 5. The whole system can be obviously miniaturized as single components to be applied directly in a shunt oil container.

The just described device can be generally defined as "SPR sensor", i.e. a system in which the surface plasmon resonance principle is used to detect the response variation which shows the analyzed species to the light stressing according to the variation of its optic properties. In the preferred embodiment of the invention, which is absolutely not limiting, for exciting a plasmon at the metal-dielectric interface, it was chosen to use the ATR method (Attenuated Total Reflection) in the Kretschmann geometry. In fact, many experimental studies have shown that the optic exciting with the ATR method guarantees that the SPR sensor is more sensible to the changes in the optic properties of the dielectric than other exciting systems such for example the ones with diffraction grating. Moreover, in the Kretschmann configuration the metal film is in direct contact with the dielectric to be monitored.

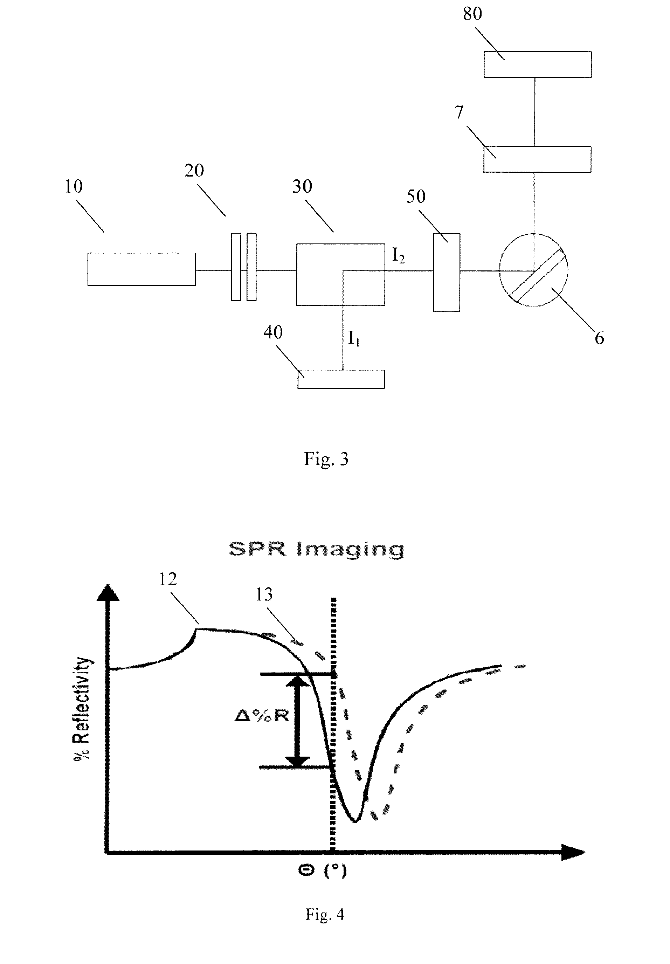

In fig. 3 it is shown a detail of the path covered by the light beam from the emission point to the plasmon stressing point. For example, in a preferred, absolutely not limiting embodiment of the invention, the light source 10 can be made up of a laser He-Ne (power 1 mW) having a wave length equal to 632 nm in the visible field. Said laser 10 passes through two neutral optic filters 20 which reduce its intensity in order to avoid heating effects localized in the metal film and the possible photodiode saturation. In order to eliminate the undesired phenomenon of the fluctuation of the emission intensity from the laser source and to avoid that this one influences the acquired piece of information, altering it, the laser beam 10 is made to pass through a "beam separator", the so called beam splitter 30, thus obtaining two distinct beams of equal intensity. The first one thereof is the referring beam and is directed to a photodiode 40, while the second beam goes to excite the plasmon wave, passing through a linear polarizer 50. Such polarizer is adjusted so that a type p polarization is obtained with respect to the incidence plane at the base of the prism. Once gone out from the polarizer 50, the light beam reaches the optic system made up of the prism 6, which is integral with a motorized rotor which allows the going out beam to center the second photodiode or detector 7. For measuring the current associated to both the beams it is used a picoammeter. Connected to the DC motor, the motorized rotor, on which it is anchored the prism 6, allows the incidence angle to be scanned and interfaced to a data processing system by means of a suitable control card. As realizing technique it can be chosen for example to detect the resonance curves by means of angular interrogation. These ones are obtained measuring the ratio between the reflected light intensity I₂ and the reference beam intensity I₁, according to the incidence angle θ of the beam at the base of the prism, around the resonance angle. One individuated the incidence angular interval in which the plasmon exciting is to be expected by using a dedicated simulation program, it can be detected the whole resonance curve by a scansion in a more significant angular interval. Once crossed the prism 6 and stricken the metal film, the beam undergoes a total reflection going out from the prism side opposed to the impact one. Therefore, the light intensity of the reflected beam strikes the wide silicon photodiode 7. The current is measured by a second picommeter interfaced to the data processing system. From the sample-prism system, the laser beam reaches the preamplifier SPR and the data acquiring card of the data processor, for example a personal computer 80. The data can be processed by means of a suitable software which allows the plasmon resonance curves (R, θ) to be acquired.

Between the base of the prism and the metal film it is provided an optic contact by interposing therebetween a very thin layer of "matching fluid" (for example ethylene glycol having a refraction index assigned at a specific wave length) which connects the refraction indexes of the substrate and prism. The metal films can be deposited on a glass substrate for example by means of the thermal evaporation technique and the thickness is accurately controlled during the deposition.

The dielectric medium, made up of the oil and/or the fuel to be analyzed, has to be put in contact with the metal film by means of a suitable system, such for example a rotating pump. Therefore, the plasmon resonance curves are detected measuring the I₂ intensity of the reflected light according to the angle θ of the incident beam at the base of the prism and the intensity of the reference beam I₁. The ratio R = I₂/I₁ provides the reflectance R according to the incidence angle. If R is equal to one hundred, it means that the incident beam is completely reflected, since I₂>>I₁. In this case no fading wave is able to stress the plasmons, and so, there is no plasmon resonance. When instead R tends to zero, it means that there is the maximum plasmon resonance. Once individuated the resonance angle, the beam is lead to an incidence angular position corresponding to the maximum slope of the resonance curve, i.e. corresponding to an angle θf slightly lower than the resonance one, in the linear portion of the plasmon resonance curve.

At this point, the angle being fixed, it is possible to determine the intensity of the reflected light according to the time. The thus obtained curves are the dynamic responses of the SPR sensor. Concerning the body of the sensing device which uses the SPR technique, it consists of a metal film (about 50 nm) generally belonging to the group 11 (noble metals) of the Periodic Table of the Elements, deposited on a transparent optic substrate by means of thermal evaporation. The most commonly used metals are Ag, Au, Ti, Cr, Cu, Al and In since they have the capacity to withstand the surface plasmon waves, i.e. they have free electrons in the conduction band able of resonance with the incidence light at a suitable wave length. Therefore the laser intensity should be able to overcome the energy gap between the valence band and the conduction band and to make the conduction electrons move thus generating plasmons. The metal film is coupled to a glass prism. Among all the metals of the group 11, silver and gold are the most commonly used to withstand the surface plasmon, since the other metals are too reactive or too oxidation susceptible, and can alter the observation. Instead, in the sensing gas applications, since gold and silver are not reactive with many gases, the surface is coated with a highly reactive metal oxide or a thin polymer film so that the induced reaction between these ones and the gases determines changes in the optic properties of the metal layer needed for measuring. The critical angle has physical origin and comes directly from the Snell law and at such an angle there occurs the attenuated total reflection. On the other hand the SPR phenomenon depends on the dielectric constant of the deposited metal.

In the light of what said so far, the new device object of the present invention allows the motor oil (or fuel) to be analyzed in an internal combustion engine, by estimating the refraction index of the liquid to be analyzed in the chamber where it flows. Referring to fig. 4, if in the oil (or fuel) there is a variation of refraction index, the light signal 5 reflected which reaches the detector 7 (after detection and processing) varies from the black curve 12 to the curve 13 in broken line, once the incidence angle θ of the light beam is fixed.

Therefore, after a suitable setting and calibration of the apparatus, the system can be used to observe different fluids, which for example are degraded in time with consequent variation of their optic characteristics, or fuel mixture present in the tank. As starting data there are the materials and the thickness of the metal film, the dielectric medium (refraction index), the glass prism and the light source (intensity, wave length, etc...) of the sensing SPR device: it can be determined the angle θₛₚᵣ of the reflectance curve. At this point the only parameter not known is the refraction index of the dielectric, i.e. the fluid to be analyzed. So, once the sensor sensibility is established, i.e. the speed with which the resonance angle varies while the refraction index in the SPR device varies, it is possible to establish the refraction index corresponding to a determined resonance angle.

The whole system can be obviously miniaturized as a unique component to be applied directly on a shunt of the pipe containing the lubricant before it reaches the circuit to which it is destined.

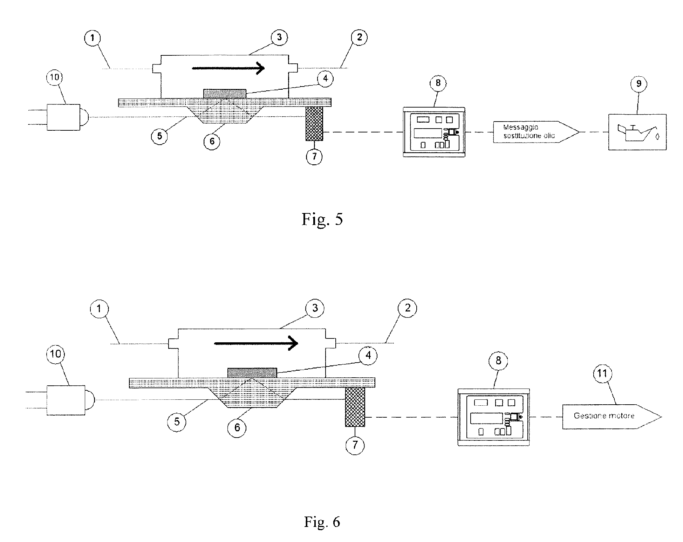

Such a preferred embodiment of the device is schematized in fig. 5: it shows that the lubricant, withdrawn from the motor oil circuit, flows from the inlet section 1 of the analyzer 3 to the outlet section 2. Under the analyzer 3, a monochromatic light beam 5 generated by a laser source 10 strikes on the metal surface 4 after passing through an optic prism 6 according to a specific incidence angle; the device is provided with a detector 7 realized so that it detects the reflected light beam in this incidence angle. A control unit 8 records the curve detected by the sensor and sends (if necessary) the oil substituting signal to the control panel 9 of the vehicle. The electronic portion of the device object of the present patent was developed to amplify and filter the reflected light signal coming from the laser source 10 and to communicate with the control logic unit 8. In this schematic representation for representation easiness there are not shown some details described previously in fig. 3 which refers to the emission path of the light beam 10 and its arriving to the detector 7.

The second development hypothesis of the present patent, shown in fig. 6, differs from the first preferred embodiment (fig. 5) substantially for two reasons: first of all the liquid analyzed in the analyzer 3 is fuel and not lubricating oil and, also, because the signals recorded by the control unit 8 are sent to the engine management system 11 for a following processing. As yet previously said, the same device intended for the real time monitoring of lubricating oil in an internal combustion engine, after different setting and calibration of the device, can be used for real time monitoring of the fuel of an internal combustion engine.

Clearly the aim is different from the case of the engine lubricating oil. In case of engine lubricating oil, the aim is to obtain a real time monitoring of its physical properties to intervene for its substitution when the same oil has lost its physical properties. In case of fuels, by means of the same device just described in the case of lubricating oil and by means of the same surface plasmon resonance principle it is aimed at obtaining a real time recognition of the component mixture of which the fuel is generally made up. From the real time analysis obtained by means of the device object of the present invention, the control system 8 can process the optimal configuration of the functioning parameters of the engine so that the most possible efficient functioning is guaranteed of the same engine according to the detected fuel mixture. For example, in case of multifuel fuels, in many countries of the world, it is provided by law that a minimum fraction of bio-fuel is used in the traditional fuels. This fraction can vary from country to country according to the rules in force, therefore an internal combustion engine can be in its functioning condition to be supplied by a fuel made up of different component mixtures. Surely this working conditions are not the optimal ones for which the same combustion engine is designed. The device according to the present invention, as it is widely described in the case of the engine lubricant is able to analyze and recognize in real time the characteristics of the component mixture of the fuel and to transmit the result to the engine control unit 8, through which the functioning parameters characteristic of the internal combustion engine can be modified (for example the ones relative to the fuel injection in the engine), trying to optimize the global yield.

## Claims

1. Method for real time monitoring of working fluids in internal combustion engines, based on the surface plasmon resonance (SPR) comprising the following steps:
a. emission of an electromagnetic wave (5) in the visible and/or infrared spectrum;
b. detection of an electromagnetic wave reflected by an optic prism (6) - metal film (4) and dielectric, this latter comprising the working fluid;
c. transduction of the reflected electromagnetic wave in a physical-chemical parameter of the working fluid;
d. processing of the physical-chemical parameter values;
e. interaction with the engine control system (8) for signaling possible anomalies or for the request of modifications of the engine management.

2. Method according to claim 1, **characterized in that** said working fluids are fuels and/or lubricants.

3. Method according to claim 1 or 2, **characterized in that** said emission of electromagnetic wave (5) is a laser source (10).

4. Method according to any one of claims 1 to 3, **characterized in that** the detection of the electromagnetic wave (5) occurs by interposing a plurality of detectors (40, 7) between the emission source (10) and the data processor (80).

5. Method according to any one of claims 1 to 4, **characterized in that** said transduction of the detected electromagnetic wave (5) in a corresponding variation of the physical chemical parameter of the fluid to be analyzed occurs by estimating the refraction index of the dielectric in the analyzer (3).

6. Method according to the preceding claim, **characterized in that** said estimation of the refraction index of the dielectric occurs by means of the detection of the resonance curves (12, 13) by means of the angular interrogation technique.

7. Method according to any one of claims 5 or 6, **characterized in that** said resonance curves are detected measuring the I₂ intensity of the reflected light according to the angle θ of the incident beam at the base of the prism and the intensity of the reference beam I₁, wherein the ratio R = I₂/I₁ provides the reflectance R according to the incidence angle θ.

8. Method according to any one of claims 1 to 7, **characterized in that** said processing consists in the determination of the θₛₚᵣ angle of the reflectance angle and in the following determination of the refraction index of the dielectric, i.e. of the fluid to be analyzed.

9. Method according to any one of claims 1 to 8, **characterized in that** a control unit (8) records the curve detected by the sensor and sends the signal of anomaly to the control panel of the vehicle.

10. Method according to any one of claims 1 to 8, **characterized in that** a control unit (8) records the curve detected by the sensor and modifies consequently the control parameters of the injection of fuel in the engine.

11. Device for real time monitoring of the working fluids in internal combustion engines having characteristic fuels and/or lubricants, based on the physical surface plasmon resonance principle (SPR) according to the method as claimed in one or more preceding claims, comprising a coupling between an optic system (6) and a metal layer (4), a transduction system of the signal between variation of the optical property and variation of the property of interest, an electronic system for processing said data, said device being **characterized in that** the analyzer (3) containing the working fluid to be analyzed is put directly in contact with the coupling optic system (6)- metal layer (5).

12. Device according to claim 11, **characterized in that** said optic system is made up of an electromagnetic radiation source (10) and an optic prism (6).

13. Device according to any one of claims 11 to 12, **characterized in that** said metal film (4) belongs to the group eleven of the Periodic Table of the Elements (noble metals).

14. Device according to any one of claims 11 to 13, **characterized in that** said metal film (4) is deposited on the transparent optic substrate (6) by means of thermal evaporation.

15. Device according to any one of claims 11 to 14,
**characterized in that** the thickness of the metal film (4) is approximately in the range between 40 nm and 80 nm.
